(19) Europäisches Patentamt
European Patent Office
Office européen des brevets

(11) EP 2 374 448 A1

(12) **EUROPEAN PATENT APPLICATION**

(43) Date of publication:
**12.10.2011 Bulletin 2011/41**

(21) Application number: **10003705.0**

(22) Date of filing: **06.04.2010**

(51) Int Cl.:
***A61K 9/00*** (2006.01) ***A61K 9/70*** (2006.01)
***A61K 31/422*** (2006.01) ***A61K 47/18*** (2006.01)
***A61K 47/20*** (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(71) Applicant: **Labtec GmbH**
**40764 Langenfeld (DE)**

(72) Inventors:
- **Breitenbach, Armin, Dr.**
  **51371 Leverkusen (DE)**
- **Schwier, Nina, Dr.**
  **41515 Grevenbroich (DE)**

(74) Representative: **Von Renesse, Dorothea et al**
**König Szynka Tilmann von Renesse**
**Patentanwälte Partnerschaft**
**Lohengrinstrasse 11**
**40549 Düsseldorf (DE)**

(54) **Oral film formulation**

(57) Pharmaceutical composition comprising at least one triptan and at least one thiol-group containing reducing agent.

**Figure 2**

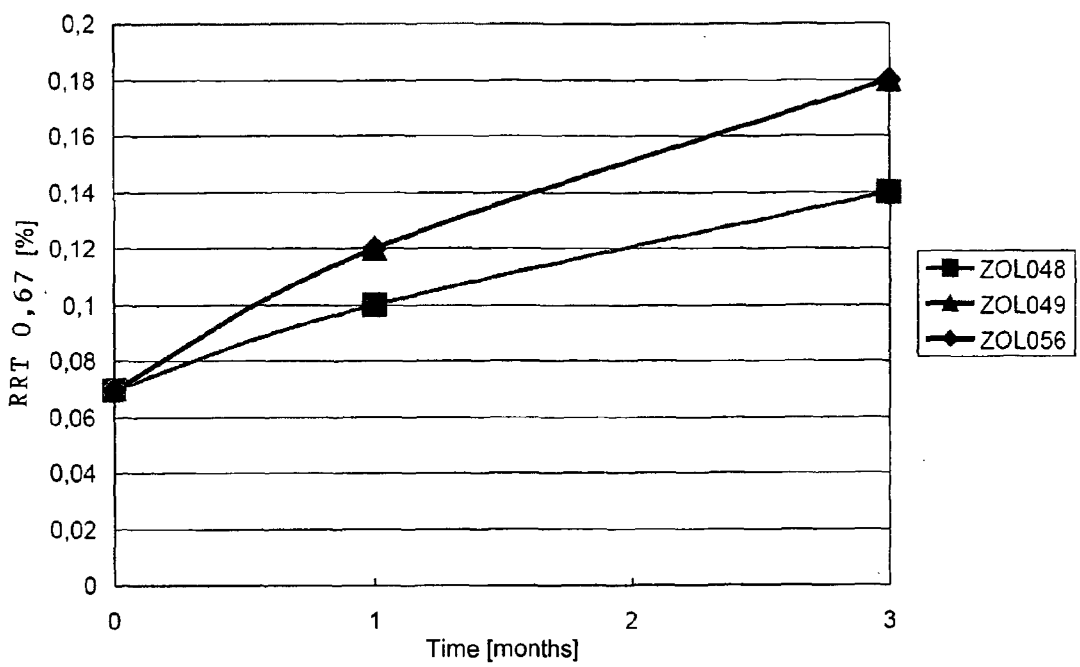




Printed by Jouve, 75001 PARIS (FR)

**Description**

FIELD OF THE INVENTION

The invention relates to an oral film formulation comprising an indole serotonin receptor antagonist (triptan), its production and use for the treatment of headache.

BACKGROUND OF THE INVENTION

Pharmaceutical dosage forms, such as orally dissolvable film formulations, which are dissolved rapidly in the mouth, are advantageous in a wide variety of aspects; in particular they facilitate the oral administration of medicaments to patients who have problems in swallowing or suffer from nausea or emesis. For these patients swallowing conventional oral administration forms such as film-coated tablets is often cumbersome. Due to the rapid disintegration of the film formulation within the buccal cavity the active ingredient from film formulation can be incorporated either over the oral or gastric mucosa. Accordingly these films can be designed to enable or support either gastrointestinal or buccal adsorption of the active ingredient.

Headache is a common symptom of various conditions which all result in pain of the pain sensitive structures of the head. The World Health Organization (WHO) estimates that one adult in 20 has a headache every or nearly every day. The most relevant condition is migraine. Approximately 240 million people have migraine attacks each year, with women being affected three times more often than men. In addition, in developed countries, tension type or "stress" headaches are estimated to affect two-thirds of all adult males and over 80% of adult females.

Headache disorders may impose substantial hardships and burdens on the afflicted individuals including personal suffering, impaired quality of life and high financial cost. Social activity and work capacity are reduced in almost all migraine sufferers and in 60% of tension headache sufferers. Finally, the long-term effort of coping with chronic headache disorders may also predispose the individual to other illnesses, such as depressions.

Triptans which represent modified forms of serotonin (5- hydroxytryptamine; 5-HT), have been developed for the treatment of migraine headaches. Triptans are serotoninergic agents that exhibit receptor-selective properties. Although the principal mechanism of action of triptans is still under research, it is understood that they relieve various symptoms of a migraine headache by inhibiting the over activity of trigeminal nerve terminals through serotonin 5-HT1 B, 5-HT1 D, 5-HT1 F receptors that exist in blood vessels in the brain and trigeminal nerves. Furthermore they inhibit inflammation around blood vessels, hyperlucency and vasodilation.

Triptans are currently regarded as the standard therapy of migraine. They are commercially available in various administration forms, such as e.g. the tablet Zomig-ZMT® comprising zolmitriptan. From WO 2008/040534 A2 non-mucoadhesive oral films with triptans are known. Oral triptan dosage forms are also disclosed in WO 2009/014960 A1.

It has now been found by the inventors of the present invention that within oral films the chemical stability of triptans often is unsatisfying. It particular it was observed that within oral films triptans to a certain extend degrade or metabolize to their N-oxide or other unwanted degradation products, which are pharmacologically ineffective. These degradation products or metabolites impede the quality of the triptan containing pharmaceutical composition.

Thus, it is the objective of the present invention to provide a pharmaceutical composition containing a triptan, in particular a film formulation, wherein the triptan exhibits an increased chemical stability.

SUMMARY OF THE INVENTION

The invention relates to a pharmaceutical composition comprising a pharmaceutically effective amount of at least one triptan and at least one thiol-group containing reducing agent. This thiol-group containing reducing agent exhibits the function of an antioxidant agent, which stabilises the chemical integrity of the triptan. The pharmaceutical composition preferably is an oral film formulation. Accordingly the invention also relates to an oral film formulation, which comprises a pharmaceutically effective amount of at least one triptan, at least one film-forming polymer and at least one thiol-group containing reducing agent. The invention in particular relates to pharmaceutical compositions, e.g. oral film formulations, containing zolmitriptan.

The invention is based on the finding that in the presence of at least one thiol-group containing reducing agent the chemical stability of the indole serotonin receptor agonist within a pharmaceutical composition is increased. This in particular applies when the indole serotonin receptor agonist is presented in an oral film formulation. With other words: At the presence of a thiol-group containing reducing agent the total amount of triptan degradation products or metabolites within the composition is reduced compare to compositions in absence of thiol-group containing reducing agent. In particular the total amount of the N-oxide of the respective triptan is reduced compared to an identical triptan containing pharmaceutical composition without a thiol-group containing agent.

The N-oxide is a well known degradation product of triptans, which all are chemically characterised in containing

an alylamino-group. The oxidation of this group results in the respective N-oxide. The N-oxide of zolmitriptan is known as "zolmitriptan N-oxide or (4S)-4-[[3-[2-(Dimethyloxidoamino)ethyl]-1H-indol-5-yl]methyl-2-oxazolidinon (CAS No 251451-30-6).

Accordingly the invention relates to a pharmaceutical composition comprising a stabilized triptan, e.g. stabilized zolmitriptan, namely to a composition wherein the zolmitriptan is stabilised with a thiol-group containing reducing agent against degradation into its N-oxid. The stabilising effect of the thiol-group containing agent does not only result in a reduced degradation of zolmitriptan into its N-oxid, but extends also to a so far unknown further degradation products, which the inventors designated "RTT 0.67" (according to its retention time in a chromatography).

The invention furthermore relates to a method of stabilising triptans in a pharmaceutical composition and to the use of thio-group containing reducing agents for the stabilisation of triptans in pharmaceutical composition, in particular in pharmaceutical oral films.

DEFINITIONS

As used throughout the present application the following terms have the meaning indicated unless otherwise indicated:

The term "disintegrate" as used herein is defined as in chapter 701 of the U.S. Pharmacopoeia (2005 USP/NF) for uncoated tablets, using a basket rack assembly operating at 30 cycles per minute through a distance of 5.5 cm, in a disintegration medium at 37°C. When disintegration requirements are discussed herein, they are preferably met under the foregoing testing conditions, at a pH of 4.0 or 6.8. A film or other dosage form is said to be "disintegrated" if it is completely disintegrated, a state in which any residue of the unit remaining on the screen of the test apparatus, or in the mouth, is a soft mass having no palpably film core, or fragments of a tablet coating or capsule shell. Disintegration thus does not require the complete dissolution of the dosage unit or the active agent, although a dissolved dosage unit would typically be completely disintegrated.

"Thiol-group containing reducing agent" refers to a chemical substance that contains at least one -SH group (thiol group) and is able to reduce another species in a redox (reduction-oxidation) reaction. Examples include but are not limited to glutathione, cysteine, methionine, thioglycerol, thiogycol acid and others.

The term "triptan" is used interchangeably with "indole serotonin receptor agonist" and refers to an agent that binds to one or more of a 5-HT1 B receptor, a 5-HT1 D receptor, and a 5-HT1 F receptor and effects vasoconstriction of cerebral blood vessels and/or inhibition of pro- inflammatory neuropeptide release. An indole serotonin receptor agonist comprises an indole-3-alkylamine structure. Representatives of this class of compounds are e.g. almotriptan, eletriptan, frovatriptan, naratriptan, rizatriptan, sumatriptan or zolmitriptan.

The term "stability" as applied to the active ingredient is the capacity of the active ingredient within the pharmaceutical composition (here: the triptan compound) to remain within the established specifications, i.e. to maintain its identity, strength, quality and purity throughout the retest or expiration dating period. As applied for the instant invention the stability is assessed in an accelerated stability study of the final pharmaceutical composition (the drug product), namely the pharmaceutical composition is subjected to stress conditions of 60°C over a period of 15 days.

In the context of the invention the term "degradation product", which is used interchangeable with "metabolites" of the triptan relates to any molecular compound resulting from changes of the triptan brought about over time and/or by the action of, e.g., light, temperature, pH, water, or by reaction with any other compound in the composition. Such changes may occur as a result of manufacture and/or storage or metabolisation (e.g., deamidation, oxidation, aggregation, proteolysis) of the triptan containing composition.

As used herein, an "antioxidant" or "anti-oxidant agent" refers to any molecule capable of slowing down or preventing the oxidation of other molecules.

"Headache" refers to any kind of pain, where pain sensitive structures of the head are involved. "Headache" in particular encompasses migraine headache, cluster headaches, rebound headaches, and status migrainosus. "Migraine headache" refers to a subset of headaches characterized by unusually severe, unilateral, throbbing, headache pain, usually persisting for 4 hours to 72 hours, and often including one or more of the following symptoms: nausea, vomiting, sensitivity to light or sound.

The term "treatment" or "treating pain" refers to administration to an individual of an agent of interest wherein the agent alleviates, reduces, mitigates or prevents any pathology or disorder of a subject (in particular a human) suffering therefrom. The term "treatment" therefore includes prophylaxis and prevention of disorders.

The term "pharmaceutically acceptable salts" is used to describe those salts in which the anion (or cation) does not contribute significantly to the toxicity or pharmacological activity of the salt, and, as such, they are the pharmacological equivalents of the bases of the compounds to which they refer. Examples of pharmaceutically acceptable acids that are useful for the purposes of salt formation include but are not limited to hydrochloric, hydrobromic, hydroiodic, citric, acetic,

benzoic, mandelic, fumaric, succinic, phosphoric, nitric, maleic, mucic, isethionic, palmitic, tannic and others. The active salt combinations of the pharmacologic ingredients may be the free acids, bases or as salts having anionic functional groups such as bitartrate, maleate, citrate, chloride, bromide, acetate and sulfate. The source of the functional groups may be natural or synthetic.

"Pharmaceutically acceptable carrier" or "suitable carrier" refers to a carrier that is conventionally used in the art to facilitate the storage, administration, and/or the healing effect of the agent.

"Therapeutically effective dose", "therapeutically effective amount" or "an effective amount" refers to an amount of an active ingredient that is useful for treating migraine, cluster headache, headache or altitude sickness.

"Tmax" as used herein, means the time to the maximum observed plasma concentration.

The term "non-mucoadhesive" or "non-mucoadhesive dosage form" means that the dosage form does not adhere to the mucosal surfaces of the buccal cavity as an intact film or disintegrated film residue.

The term "film" relates to any two-dimensional layered or plane structure. A "film formulation" is any composition which is capable of forming a film. The film can be prepared fro the film formulation by various methods, including drying or curing. A "film-forming polymer" refers to a polymer that can form a continuous film upon evaporation of the solvent or carrier and/or upon cure of the polymer.

The term "plasticizer" as used herein is meant to designate a component or a mixture of components having a plasticizing action on the film.

Unless specified otherwise, the term "wt. %" as used herein relates to the relative amount by weight with respect to the final (dried) pharmaceutical product (as opposed to the composition which is used for providing it). It this denotes the percentage of the total dry weight contributed by the respective component.

DETAILLED DESCRIPTION OF THE INVENTION

As outlined above the invention relates to a triptan containing pharmaceutical composition with an increased stability of the triptan, i.e. a reduced content of triptan degradation products. The stability of triptan is increased by the presence of a thiol-group containing agent. In one embodiment of the invention the concentration of the thiol-group containing agent in the pharmaceutical composition is about 0.01 to about 7 wt %. Preferably the thiol-group containing agent is present between 0.07 and 6 wt %. In a more preferred embodiment the concentration of the thiol-group containing reducing agent is between 0.2 and 1.0 wt%. In one embodiment the concentration is not more than 0.5 wt.%.

According to the invention the relative amount of triptan degradation products with respect to the original total amount of triptan can be reduced to a maximum of about 1 %, i.e. at least 99 % of the triptan can be recovered. This applies after the pharmaceutical composition has been subject of an "stress test", namely an accelerated stability study (15 days storage at 60°C; as exemplified in example 1 *infra).* In a preferred embodiment of the invention the relative amount of triptan degradation products within the pharmaceutical composition after the stress test has a maximum of 0.9% or even 0.8 %. In a most preferred embodiment the amount of degradation products is below 0.75% (all this data relate to an assessment of the degradation products after the stress test).

As outlined above the increased stability achieved by the invention in particular relates to the N-oxids of the triptans. Accordingly the invention provides a pharmaceutical composition, preferably an oral film, which contains a maximum of 1 % of a triptan N-oxide, in particular a maximum of 0.8%, more preferred a maximum of 0.5 %, most preferred a maximum of 0.2% of the triptan N-oxide.

The triptan used in the pharmaceutical composition according to the invention can be, but are not limited to, the indole serotonin receptor agonists of the following formula I:

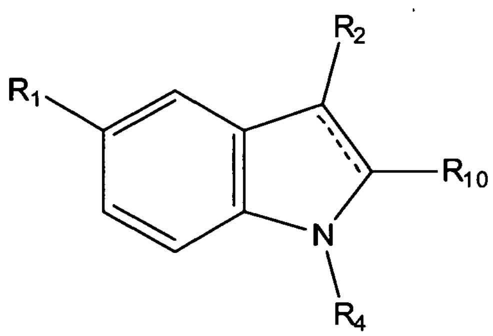

(I)

wherein $\mathbf{R_1}$ is

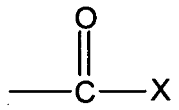 ,

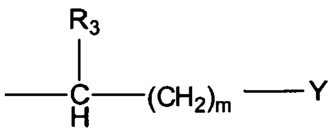 , 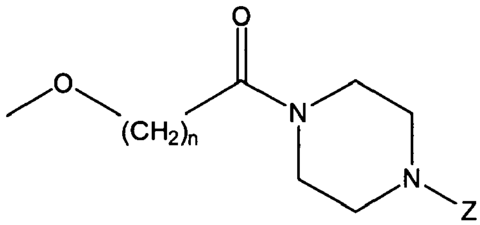

wherein Y is

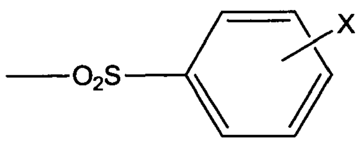 , 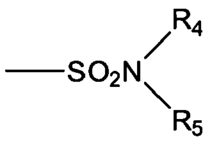

or a 5- or 6-membered cycloalkyl, wherein in some embodiments 1, 2, or 3 $CH_2$ groups are replaced by 0, S, or NH, which cycloalkyl will in some embodiments substituted with an oxo group;
X is H, C1-3-alkyl, C1-3-alkoxy, halogen, $CF_3$, $NO_2$ or $NH_2$;
$R_3$ is H or C1-3-alkyl;
$R_4$ is H, C1-6-alkyl or C3-6-alkenyl;
$R_5$ is H, C1-3-alkyl, C3-6-alkenyl, aryl, C1-4-alkylene or C5-7-cycloalkyl;
R4 and R5 together form the group $(CR_3)_s$ with s = 2, 3, 4, 5 or 6;
Z is C1-C6 alkyl, C3-C6 alkenyl, C1-4-alkylene or C5-7-cycloalkyl, aryl or heteroaryl,
or Z is

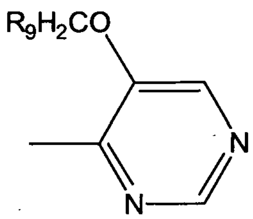 , 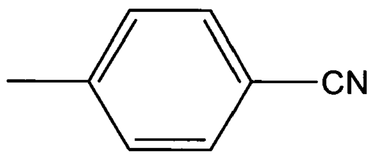

wherein $R_2$ is

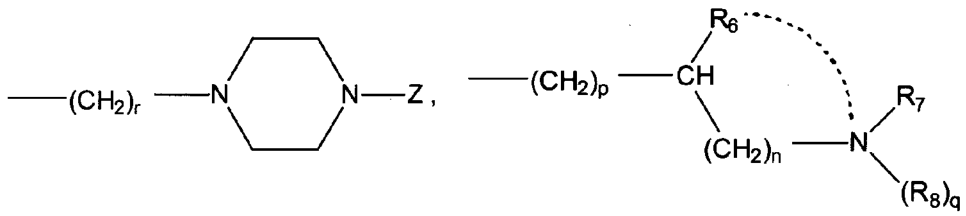

$R_2$ and $R_{10}$ together form the group $(CR_3)_s$ with s = 2, 3, 4, 5 or 6, substituted with $NHR_9$
$R_6$ is H or (CH2)r;
$R_7$ and $R_8$ are the same or different, and are each independently H, or C1-3-alkyl;
$R_9$ is H, C1-6-alkyl, or C3-6-alkenyl;
m, n, and r may be the same or different and are each independently an integer from 0 to 3, e.g., are each independently 0, 1, 2, or 3;
p is an integer that is 0 or 1; and
q is an integer that is 0 or 1;
with the proviso that when $R_6$ is $(CH_2)_r$ and r is not zero, this group can be bound to the nitrogen atom of the radical $NR_7(R_8)_q$ by a single bond, in which case q is zero. In some embodiments, the indole serotonin receptor agonist is a physiologically acceptable salt of a compound of Formula I, or a solvate of a compound of Formula I, or a pro-drug of a compound of Formula I. In some embodiments, e.g., the agonist is a succinate salt of a compound of Formula I.

The triptans of the invention are preferably selected from the group consisting of zolmitriptan sumatriptan, eletriptan, rizatriptan, naratriptan, almotriptan, frovatriptan, avitriptan and donitriptan. zolmitriptan is particularly preferred.

Zolmitriptan is chemically designated as *(*S)-(4)-[[3-[2-(dimethylamino)ethyl]-1*H*-indole-5yl] methyl]-2-oxazolidinone, and has the following chemical structure:

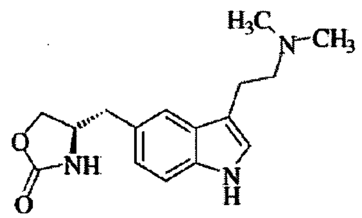

Sumatriptan (Imitrex®) is chemically designated as 3-[2-(dimethylamino)ethyl]-N-methyl-1*H*-indole-5-methanesulfonamide. Sumatriptan is represented by the following chemical structure:

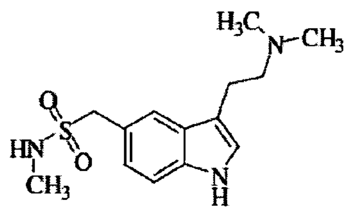

Sumatriptan can be administered as any pharmaceutically acceptable salt that demonstrates adequate stability upon storage and bioavailability upon administration, but a preferred form of sumatriptan for purposes of this invention is sumatriptan succinate (1:1 ).

Eletriptan is chemically designated as (R)-3-[(l-Methyl-2- pyrrolidinyl)methyl]-5-[2-(phenylsulfonyl)ethyl]-1*H*-indole, and is represented by the following chemical structure:

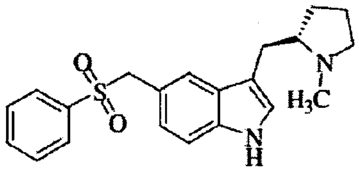

Eletriptan can be administered as any pharmaceutically acceptable salt that demonstrates adequate stability upon storage and bioavailability upon administration, but a preferred form of eletriptan for purposes of this invention is eletriptan monohydrobromide.

Rizatriptan is chemically described as N,N-dimethyl-5-(IH-1,2,4-triazol-l- ylmethyl)-1H-indole-3-ethanamine. Rizatriptan is depicted by the following chemical structure:

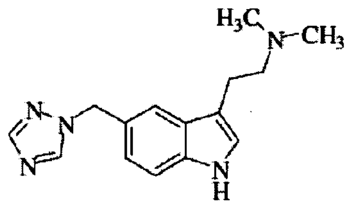

Rizatriptan can be administered as the base or as any pharmaceutically acceptable salt that demonstrates adequate stability upon storage and bioavailability upon administration, but a preferred form of rizatriptan for purposes of this invention is rizatriptan benzoate.

Naratriptan is chemically designated as N-methyl-3-(1-methyl-4-piperidinyl)-1*H*-indole-5-ethanesulfonamide, and has the following chemical structure:

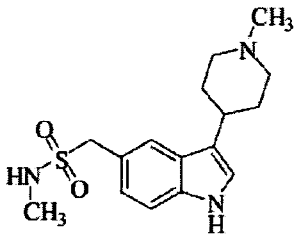

Naratriptan can be administered as the base or as any pharmaceutically acceptable salt that demonstrates adequate stability upon storage and bioavailability upon administration, but a preferred form of naratriptan for purposes of this invention is naratriptan hydrochloride.

Almotriptan is chemically designated as I-[[[3-[2-(dimethylamino)ethyl]-1*H*-indole-5-yl]methyl]sulfonyl]pyrrolidine and has the following chemical structure:

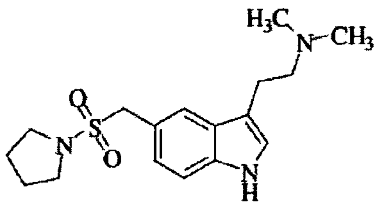

Almotriptan can be administered as the baseor as any pharmaceutically acceptable salt that demonstrates adequate stability upon storage and bioavailability upon administration, but a preferred form of almotriptan for purposes of this invention is almotriptan maleate.

Frovatriptan is chemically described as (+)-(*R*)-3-methylamino-6-carboxamido-1,2,3,4-tetrahydrocarbazole. Frovatriptan is depicted by the following chemical structure:

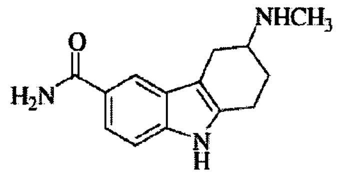

Frovatriptan is preferably administered as the succinic acid salt.

Avitriptan is chemically designated as 4-(5-Methoxy-4-pyrimidinyl)-1-[3-[5-[[(methylamino)-sulfonyl]methyl]-1*H*-indole-3-yl]propyl]piperazine, and has the following chemical structure:

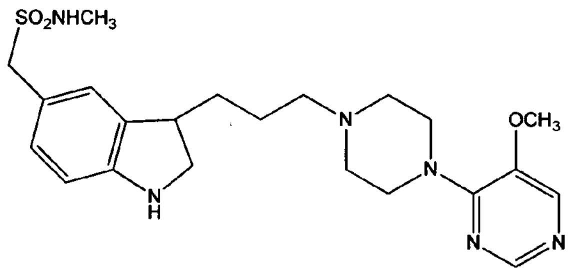

Donitriptan is chemically designated as [4-[4-[2-[3-(2 amino-ethyl)-1*H*-indole-5-yloxy]-acetyl]-piperazin-1-yl]-benzonitrile hydrochloride], and has the following chemical structure:

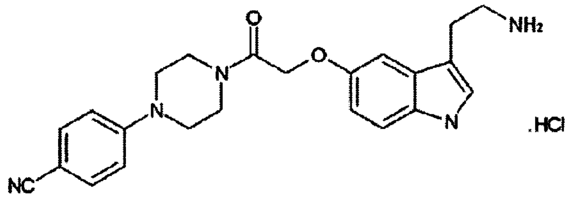

The amount of triptan per dosage form of the pharmaceutical composition provided according to the invention depends on the triptan used. If an oral film is provided thos film preferably is bioequivalent to the tablet, as assessable by its Tmax value. Preferred dosages of selected triptans and their Tmax values are listed in the following table.

| Triptan | Amount per dosage form [mg] | Tmax after oral administration [h] |
|---|---|---|
| zolmitriptan | 1.5 - 7.5 (preferably 2.5 - 5) | 1-4 (1-2 for migraine free period, 2.5-3 during attack) |
| Sumatriptan | 15 - 125 (preferably 25 -100) | 1.5 - 3 (preferably 2 - 2.5) |
| Eletriptan | 10 - 100 (preferably 10 - 60) | 1.0 - 3 (preferably 1.5 - 2.0) |
| Rizatriptan | 2.5 - 15 (preferably 5 - 10) | 0.5 - 3 (preferably 1.0 - 2.5) |
| Naratriptan | 0.5 - 5 (preferably 1 - 2.5) | 1.5 - 4.5 (preferably 2.0 - 4.0) |
| Almotriptan | 2.5 - 15 (preferably 6.25 - 10) | 0.5 - 4 (preferably 1.0 - 3.0) |
| Frovatriptan | 1.0 - 5 (preferably 2.5) | 0.5 - 4 (preferably 1.0 - 3.0) |
| Avitriptan | 25 - 150 (preferably 50 - 100) | 0.25 - 1 (preferably 0.5) |
| Donitriptan | 0.5-5 | - |

The pharmaceutical composition of the invention can comprise the at least one triptan in a concentration of 0.1 and 60 wt.%, preferably in a concentration of 0.1 to 50 wt.% and most preferably in a concentration between 5 and 20 wt.%.

The thiol-group containing reducing agent which can be applied according to the invention can be selected from the group consisting of glutathione, L-cysteine, dithiothreitol, methionine, thioglycerol, thiogycol acid, thio lactic acid or a pharmaceutically acceptable salt thereof. The use of glutathione, in particular the use of 0.2 to 1.0 wt %, or a salt or a derivative thereof as the stabilising agent is preferred.

In a specific aspect of the invention thiol-group containing reducing agent can be used whereas the thiol-group containing reducing agent is not L-glycine or cysteine.

In a further aspect of the invention the ratio of the thiol-group containing reducing agent to the triptan is below 2:1, in particular not more than 1:1, preferably not more than 0.5:1, and most preferred not more than 0.1:1.

When the pharmaceutical composition of the invention is an oral film, then a film forming polymer can be used, which is a water soluble polymer, e.g. a polyvinyl alcohol. Preferred is a polyvinyl alcohol of 5-100 KDa and a degree of hydrolysis of 85 to 90%, and most preferred is a polyvinyl alcohol of 20-40 KDa and especially preferred is Mowiol 4-88.

According to on embodiments of the invention the formulation comprises a plasticizer, which preferably is a polymer and more preferably a water-soluble polymer.

In an preferred embodiment of the invention said plasticizer is at least one polyethylene glycol polymer (PEG), more preferably a combination of at least one solid PEG and at least one liquid PEG (both at room temperature), most preferably a combination of a PEG with a molecular weight between 3000 and 6000 Da and a PEG with a molecular weight between 250 and 1000 Da, and specifically a combination of PEG 400 and PEG 4000.

In one aspect of the invention the oral film comprises starch or a starch derivative, preferably rice starch.

In a further aspect of the invention the oral film comprises one or more additives. Additives include effervescent agents, tensides, sweetening agents, flavouring agents, taste masking agents, colouring agents, colourants and filler.

In a further embodiment of the invention the film comprises the following components in the given proportions:

a) 20 to 75 wt.% film-forming polymer
b) 0.02 to 7 wt.% thiol-group containing reducing agent
c) 2 to 35 wt.% active ingredient

Preferably these components have the concentration as follows:

a) 30 to 55 wt.% film-forming polymer
b) 0.07 to 6 wt.% thiol-group containing reducing agent (preferred below 1%, most preferred below 0.05%)
c) 5 to 20 wt.% active ingredient

In a further preferred aspect of the invention the oral film comprises the thiol-group containing reducing agent as above, and the following components as follows:

a) 40 to 45 wt.% polyvinyl alcohol
b) 1 to 4 wt.% liquid polyethylene glycol
c) 2.5 to 10 wt.% solid polyethylene glycol
d) 17 to 23 wt.% starch
e) 12 to 18 wt.% additive

In one further preferred aspect of the invention the additive fraction comprises:

a) 2 to 4 wt.% sweetening agent
b) 5 to 7 wt.% flavouring agent
c) 5 to 7 wt.% colouring agent

These preferred compositions each are characterised by the above outlined low content of triptan degradation products, in particular of N-Oxide.

In one aspect of the invention the formulation provides a film which consists of a single layer whereby the layer preferably has a homogenous structure. Preferably, the film is a single layer homogeneous film. The film can have a rigid or a flexible consistency, the latter one is preferred.

In one aspect of the invention the film has a thickness between 1 and 1000 μm, preferably between 40 and 200 μm and most preferably between 80 and 100 μm. In order to avoid an unpleasant sensation in the mouth, the film thickness must not be too great.

In one aspect of the invention, the film has a weight of from about 20 to about 100 milligrams, preferably from

about 25 to about 80 milligrams. The film may vary in surface area and preferably does not exceed 8 or 7 $cm^2$ in surface area.

In one aspect of the invention the formulation provides a non-mucoadhesive orally disintegrating film, which is able to disintegrate upon contact with saliva in the buccal cavity. The film preferably disintegrates within about 10, 20, 30 or 60 seconds of administration, after it is swallowed. The prompt disintegration and swallowing of the film helps to assure gastrointestinal absorption of the dosage form.

In another aspect of the invention the formulation provides a mucoadhesive orally disintegrating film, which is able to disintegrate upon contact with saliva in the buccal cavity. The muco-adhesiveness helps to assure mucosal absorption of the dosage form.

Preferably, the single-layered film-form preparation can be substantially free of cavities, a "cavity" being understood as being a region which is filled with a fluid (a gas and/or a liquid). Such a cavity usually has a diameter of less than 100 μm. Preferably, a film-form preparation is substantially free of gas bubbles and/or cavities that contain a fluid (gas and/or liquid).

In a further aspect of the invention the film formulation can be essentially free of tensides. A film that is essentially free of tensides has a tenside concentration of less than 1 wt.% related to the complete formulation, preferably less than 0.1 wt.%, and more preferably less than 0.01 wt.%. In a preferred aspect of the invention the film is produced without addition of tensides as component. The term "tenside" as used herein refers to any substance or compound that reduces surface tension when dissolved in water or an aqueous solution, or that reduces interfacial tension between two liquids, or between a liquid and a solid. Unless otherwise indicated, the term "tenside" includes substances or compounds that are anionic, cationic, nonionic, zwitterionic and/or amphoteric in nature.

In another aspect of the invention the film formulation can be essentially free of effervescent additives. A film that is essentially free of effervescent additives has a concentration of effervescent agents of less than 1 wt.% related to the complete formulation, preferably less than 0.1 wt.%, and more preferably less than 0.01 wt.%. In a preferred aspect of the invention the film is produced without addition of effervescent additives as component. As used herein, an effervescent additive means a material or compound that is capable of rapidly releasing gas upon contact with aqueous solvents, or change in pH value or increased temperature. Effervescent additives are preferably substances that in the buccal cavity, e.g. by contact with the saliva, releases a gas such as $CO_2$.

In one aspect of the invention the film formulation can be essentially free of taste masking agents. A film that is essentially free of taste masking agents has a concentration of taste masking agents of less than 1 wt.% related to the complete formulation, preferably less than 0.1 wt.%, and more preferably less than 0.01 wt.%. In one aspect of the invention the film is produced without addition of taste masking agents as component. As used herein, a taste masking agent refers to a compound that interacts with a bad or unpleasant (e.g. bitter or sour) tasting substance thereby masking the bad or unpleasant taste.

A taste masking agent is preferably used within formulations to mask the bad taste of an active ingredient. The film formulation is preferably free of ion exchange resins, inclusion compounds formed with cyclodextrin or a cyclodextrin derivative, or coatings of the active ingredient with a layer, eg. comprising Eudragit. In a preferred aspect of the invention the active ingredient is contained within the formulation in free form and not e.g. encapsulated or enclosed.

Suitable sweeteners that can be included are those well known in the art, including both natural and artificial sweeteners. Suitable sweeteners include, e.g.:

A. water-soluble sweetening agents such as monosaccharides, disaccharides and polysaccharides such as xylose, ribose, glucose (dextrose), mannose, galactose, fructose (levulose), sucrose (sugar), maltose, invert sugar (a mixture of fructose and glucose derived from sucrose), partially hydrolyzed starch, corn syrup solids, dihydrochalcones, monellin, steviosides, and glycyrrhizin;

B. water-soluble artificial sweeteners such as the soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (acesulfame-K), the free acid form of saccharin, and the like;

C. dipeptide based sweeteners, such as L-aspartic acid derived sweeteners, such as L-aspartyl-L-phenylalanine methyl ester (aspartame) and materials described in U.S. Pat. No. 3,492,131,L-alpha-aspartyl-N-(2,2,4,4-tetramethyl-3-thietanyl)-D-alanin amide hydrate, methyl esters of L-aspartyl-L-phenylglycerin and L-aspartyl-L-2,5,dihydrophenyl-glycine, L-aspartyl-2,5-dihydro-L-phenylalanine, L-aspartyl-L-(1-cyclohexyen)-alanine, and the like;

D. water-soluble sweeteners derived from naturally occurring water-soluble sweeteners, such as a chlorinated derivative of ordinary sugar (sucrose), known, for example, under the product description of sucralose; and

E. protein based sweeteners such as thaumatoccous danielli (Thaumatin I and II).

In general, an effective amount of auxiliary sweetener is utilized to provide the level of sweetness desired for a particular composition, and this amount will vary with the sweetener selected. This amount will normally be 0.01 % to about 10% by weight of the composition when using an easily extractable sweetener. The water-soluble sweeteners described in category A above, are usually used in amounts of about 0.01 to about 10 wt.%, and preferably in amounts of about 2 to about 5 wt.%. Some of the sweeteners in category A (e.g., glycyrrhizin) can be used in amounts set forth for categories B E below due to the sweeteners' known sweetening ability. In contrast, the sweeteners described in categories B to E are generally used in amounts of about 0.01 to about 10 wt.%, with about 2 to about 8 wt % being preferred and about 3 to about 6 wt.% being most preferred. These amounts may be used to achieve a desired level of sweetness independent from the flavour level achieved from any optional flavour oils used.

The flavourings that can be used include those known to the skilled artisan, such as natural and artificial flavours. These flavourings may be chosen from synthetic flavour oils and flavouring aromatics, and/or oils, oleo resins and extracts derived from plants, leaves, flowers, fruits and so forth, and combinations thereof. Representative flavour oils include: spearmint oil, cinnamon oil, peppermint oil, clove oil, bay oil, thyme oil, cedar leaf oil, oil of nutmeg, orange oil, oil of sage, and oil of bitter almonds. Also useful are artificial, natural or synthetic fruit flavours such as vanilla, chocolate, coffee, cocoa and citrus oil, including lemon, orange, grape, lime and grapefruit and fruit essences including apple, pear, peach, strawberry, raspberry, cherry, plum, pineapple, apricot and so forth. These flavourings can be used individually or in admixture. Commonly used flavours include mints such as peppermint, artificial vanilla, cinnamon derivatives, and various fruit flavours, whether employed individually or in admixture. Flavourings such as aldehydes and esters including cinnamyl acetate, cinnamaldehyde, citral, diethylacetal, dihydrocarvyl acetate, eugenyl formate, p-methylanisole, and so forth may also be used. Generally, any flavouring or food additive, such as those described in the EU database of flavouring substances, may be used. Further examples of aldehyde flavourings include, but are not limited to acetaldehyde (apple); benzaldehyde (cherry, almond); cinnamic aldehyde (cinnamon); citral, i.e., alpha citral (lemon, lime); neral, i.e. beta citral (lemon, lime); decanal (orange, lemon); ethyl vanillin (vanilla, cream); heliotropine, i.e., piperonal (vanilla, cream); vanillin (vanilla, cream); alpha-amyl cinnamaldehyde (spicy fruity flavours); butyraldehyde (butter, cheese); valeraldehyde (butter, cheese); citronellal (modifies, many types); decanal (citrus fruits); aldehyde C-8 (citrus fruits); aldehyde C-9 (citrusfruits); aldehyde C-12 (citrus fruits); 2-ethyl butyraldehyde (berry fruits); hexenal, i.e. trans-2 (berry fruits); tolyl aldehyde (cherry, almond); veratraldehyde (vanilla); 2,6-dimethyl-5-heptenal, i.e. melonal (melon); 2-6-dimethyloctanal (greenfruit); and 2-dodecenal (citrus, mandarin); cherry; grape; mixtures thereof; and the like.

The amount of flavouring employed is normally a matter of preference subject to such factors as flavour type, individual flavour, and strength desired. Thus, the amount may be varied in order to obtain the result desired in the final product. Such variations are within the capabilities of those skilled in the art without the need for undue experimentation. In general, amounts of about 0.1 to about 30 wt % are useable with amounts of about 2 to about 25 wt % being preferred and amounts from about 8 to about 10 wt % are more preferred.

The compositions of this invention can also contain colouring agents or colourants. The colouring agents are used in amounts effective to produce the desired colour. The colouring agents useful in the present invention include pigments such as titanium dioxide, which may be incorporated in amounts of up to about 5 wt %, and preferably less than about 1 wt %. Colourants can also include natural food colours and dyes suitable for food, drug and cosmetic applications. These colorants are known as FD&C dyes and lakes. The materials acceptable for the foregoing spectrum of use are preferably water-soluble, and include Indigotine (E132), which is the disodium salt of 2,2'-Bis(2,3-dihydro-3-oxoindolyliden). Similarly, the dye known as Green No. 3 (E143) comprises a triphenylmethane dye and is the monosodium salt of 4-[4-N-ethyl-p-sulfobenzylamino) diphenyl-methylene]-[1-N-ethyl-N-p-sulfonium benzyl)-2,5-cyclo-hexadienimine]. A full recitation of all food dyes and their corresponding chemical structures maybe found in the Kirk-Othmer Encyclopedia of Chemical Technology, Volume 5, Pages 857-884, which text is accordingly incorporated herein by reference.

As fillers there can be used salts, such as carbonates, phosphates, oxides, such as e.g. Si02, especially in the form of Aerosil, or the like and/or cellulose and derivatives thereof, and also sparingly soluble sugars and sugar derivatives, such as, for example, lactose or starch derivatives such as cyclodextrins, provided they are in substantially undissolved form in the product and therefore fulfil the mechanical properties of a filler. Preferably, Si02 is used as filler. However, in a preferred embodiment of the invention the composition can be devoid of any filler.

In a further aspect of the invention the film is arranged on a carrier foil. Furthermore, the film according to the invention or the preparation according to the invention can be provided with a carrier foil made of polyethylene paper (PE paper), polypropylene foil (PP foil) or polyethylene terephthalate foil (PET foil). Preferably, the film according to the invention or the preparation according to the invention is provided on a carrier foil made of polyethylene paper (PE paper), polypropylene foil (PP foil) or polyethylene terephthalate foil (PET foil).

The invention also provides a method of making a film formulation to any of the above claims comprising the following steps:

(a) solubilising the active ingredient in a suitable solvent
(b) addition of the film-forming polymer and a plasticizer
(c) addition of at least on active ingredient
(d) thoroughly mixing and/or homogenisation
(d) applying the mixture to a suitable carrier
(e) removal of the solvent.

For the preparation of the film, the active ingredient(s) is(are) suspended or dissolved in a solvent. Alcohols or alcohol/water mixtures can be used as solvent.
After the addition of film-forming polymers, gel formers and optionally additives, such as sweeteners, flavourings, colourings and/or fillers, the mixture is homogenised. The mixture is applied to a carrier material with the aid of a suitable coating method. As carrier material there can be used, for example, PE paper or PP or PET foil. The coated carrier material is dried at from 30 to 120° C., preferably at from 30 to 70° C. The coated carrier material is then processed further to form separate films of defined area. This can be effected by punching, cutting or stamping. The films are individually packed into sachets with or without carrier foil. They can also be packed into multiple-dose containers. Prior to administration, where applicable the active-ingredient-containing film is removed from the carrier material.
The film formulation is used according to the invention for the administration of anti-migraine agents in the acute treatment of migraine attacks with and without aura, cluster headache and the like. Preferably, the film formulation comprising one or more anti-migraine agents is used to produce a medicament for the acute treatment of migraine attacks with and without aura, cluster headache and the like.
In a further aspect the film formulation can be used for the preparation of a medicament for treatment of altitude sickness.

## I. EXAMPLE

### 1. Preparation of the zolmitriptan film

Zolmitriptan films were prepared for zolmitriptan doses of 2.5 mg and 5 mg, respectively. The zolmitriptan film 2.5 mg has a size of 3 $cm^2$ and the zolmitriptan film 5 mg has a size of 6 $cm^2$. Both dosages were prepared with the same formulation composition and differ only in their size.
The composition of the dried 3 $cm^2$ and 6 $cm^2$ films is summarised in **Table 1**. In the right column depicts the weighing of all raw materials including water and ethanol for a 100kg batch, which has a solids content of about 33%. The dried films contain 0 to 4% residual water (relating to the weight of the dried film).

### 2. Stability analysis

### 2.1 Experimental procedures

For the stability assessment the films were subjected to an accelerated stress conditions as follows. The zolmitriptan films were incubated in a cabinet dryer for 15 days at 60°C whereby the films were kept in an unsealed petri dish. Afterwards the films were dissolved in a mixture of acetonitrile and water (50/50%) and analysed by HPLC for quantification of degradation products. Furthermore the tensile properties were determined according to ISO 527-3 and ASTM D882.
Analytical method of assay and related substances: The films are dissolved in acetonitrile/water (50/50). The solutions are analyzed by means of RP-HPLC and UV detection at 225 nm. An outline of the RP-HPLC method is given in **Table 2**.
The impurities are characterized by their respective retention times normalized to the retention time of zolmitriptan which is set to 1.0.
The content [mg zolmitriptan per film (RF)] is calculated according to:

$$\text{content}_{\text{assay}}\ [\text{mg/RF}] = \frac{c_{\text{sample}} \cdot D}{1000}$$

$C_{sample}$ concentration test solution in [µg/mL]

D dilution [ml]

The content [%] is calculated according to:

$$content_{assay}\ [\%] = \frac{content_{assay}\ [mg/RF] \cdot 100}{API[\%] * weight\ [mg]}$$

API content API in dry coating mass [%]

Matrix weight weight of film [mg]

Content of known and unknown impurities of zolmitriptan

Considering the response factors, unknown impurities are set to a response factor of 1.00. All impurities are calculated by a one point calibration using 0.5 % standard zolmitriptan at 225 nm. All impurities of the active ingredient are applied to zolmitriptan in [%]. Unknown signals from placebo, solvent or artefacts can be ignored.

The content of zolmitriptan impurities [mg/RF] is calculated according to:

$$content_{impurity}\ [mg/RF] = \frac{C_{sample}\ [\mu g/mL] \cdot D}{1000} \cdot R$$

$C_{sample}$ concentration of test solution [μg/mL]

DF dilution [ml]

R Response factor

Stage IV = 1.57

n-Oxid = 1.37

Amine Impurity = 2.11

Unknown impurities = 1.00

The content of zolmitriptan impurities [%] is calculated according to:

$$content_{impurity}\ [\%] = \frac{content_{impurity}\ [mg/RF] \cdot 100}{content\ [mg]}$$

**2.2 Product related degradation products of zolmitriptan**

The degradation of zolmitriptan resulted in formation of the zolmitriptan N-oxide and a yet unknown degradation product, which was denominated RTT 0.67 (=retention time of 0.67 hours) based on its elution characteristics. The quantification of these two degradation products was used to study several stability-improving strategies.

**2.2.1 Characterisation of the degradation product RTT0.67**

The unknown degradation product RTT0.67 is specified by the analytical procedure described in chapter 2.1 and outlined in **Table** 2.

### 2.3. Analysis of different stability strategies

In stress tests the following variations of the zolmitriptan film were analysed for their effect on zolmitriptan stability and film stability:

- ➢ Addition of HPMC
- ➢ Addition of β-cyclodextrin
- ➢ Packaging under nitrogen
- ➢ Analysis of 11 different antioxidants

#### 2.3.1 Results

A film that was prepared using hydroxypropylmethylcellulose (HPMC) as a film forming polymer exhibited increased mechanical stability of the film. However, the film exhibited increased degradation of zolmitriptan compared to the PVA-based film.

Similarly a film that contained the complexing agent β-cyclodextrin exhibited increased mechanical stability while showing decreased chemical stability of zolmitriptan.

In a further experiment the films were packaged and stored under nitrogen as protective gas atmosphere. However, this measure yielded only a slight improvement in the chemical stability of zolmitriptan.

The analysis of 11 different antioxidants showed strong differences with regard to zolmitriptan stability (see **Table 3**):

- ➢ In the presence of tocopherol, sodium EDTA, butylated hydroxytoluene (BTH), citric acid, sodium sulfite or ascorbic acid the zolmitriptan showed decreased stability.
- ➢ The addition of sodium citrate did not change the stability of the formulation.
- ➢ Ascorbyl palmitate as stabilising agent resulted in a slight improvement of the stability, whereas the thiol group-containing reduction agents glutathione. L-cysteine and the Hydrochloride salt of L-cysteine.

#### 2.3.2 Conclusion

Only the thiol-group containing reducing agents such as glutathione and L-cysteine were able to increase the stability of zolmitriptan.

### 2.4 Comparative analysis of glutathione

The zolmitriptan film was generated with 0.2% glutathione (batch ZOL 48) or without glutathione (batch ZOL047) and stored for up to 15 days at 60°C. Afterwards the films were analysed for the amount of the degradation products zolmitriptan N-oxide and RTT 0.67.

#### 2.4.1 Results

In comparison to the basic zolmitriptan film, the addition of 0.2% glutathione (batch ZOL048) reduced the N-oxide content from 0.91 to 0.17%, the RTT0.67 content from 0.18% to 0.13% and the content of the total amount of degradation products from 1.71% to 0.72% (see **Table 4**).

#### 2.4.2 Conclusions

The addition of 0.2% glutathione leads to a considerably reduction of all known zolmitriptan degradation products even under rather harsh conditions.

### 2.5 Detailed analysis of glutathione and L-cysteine

In a follow up experiment the zolmitriptan film was generated with 0.2% glutathione (batch ZOL 48), 0.2% L-cysteine (batch ZOL049) and 0.2% glutathione together with 0.2% L-cysteine (batch ZOL056) and stored for up to 3 month at 40°C and 75% relative humidity. At T=0, 1 month and 3 month the films were analysed for the amount of the degradation products zolmitriptan N-oxide and RTT 0.67.

**2.5.1 Results**

The films containing 0.2% L-cysteine (batch ZOL049), and 0.2% glutathione together with 0.2% L-cysteine (batch ZOL056) prevented the generation of the N-oxide for the same extent and were superior to a film containing 0.2% glutathione (batch ZOL 48).

The films containing 0.2% cysteine (batch ZOL049), and 0.2% glutathione together with 0.2% L-cysteine (batch ZOL056) prevented the generation of RTT0.67 for the same extent and were inferior to a film containing 0.2% glutathione (batch ZOL 48).

**2.5.2 Conclusions**

The two tested reducing agents exhibit a complementary effect on the generation of the two degradation products: While L-cysteine is superior in preventing the generation of the N-oxide, the glutathione preferably prevents the formation of RTT0.67. A combination of glutathione and cysteine exhibits the same effect as cysteine alone.

**2.6 Analysis of different glutathione concentrations**

In a further experiment the zolmitriptan film was generated with 0.2, 0.5 and 1% of glutathione and stored uncovered at 60°C. Afterwards the films were analysed for the amount of the degradation products zolmitriptan N-oxide and RTT 0.67.

**2.6.1 Results**

At all three concentrations glutathione reduced the amount of N- oxide and also the amount of the unknown degradation product RTT 0.67 as compared to a film formulation lacking glutathione.

**2.6.3 Conclusions**

Hence a film that contains glutathione in the range of 0.2% to 1% displayed enhanced stability.

**TABLE LEGENDS**

**Table 1**: Composition of dried zolmitriptan films (3 $cm^2$ and 6 $cm^2$)

**Table 2**: Outline of the RP-HPLC method used for identification and quantification of zolmitriptan and its impurities.

**Table 3:** Stability of the zolmitriptan films containing different reduction agents after stress experiments.

**Table 4:** Stability of a zolmitriptan film with or without Glutathione

**FIGURE LEGENDS**

**Figure 1:** Concentration of N-oxide using different stabilisers

**Figure 2:** Concentration of RRT 0.67 using different stabilisers.

**Claims**

1. Pharmaceutical composition comprising at least one triptan and at least one a thiol-group containing reducing agent.

2. The pharmaceutical composition of claim 1, wherein the thiol-group containing reducing agent is present in a concentration of 0.01 to about 7 wt %, preferably in a concentration of 0.07 and 6.0 wt %, most preferred in a concentration between 0.2 and 1.0 wt%.

**3.** The pharmaceutical composition of claim 1 or 2, wherein the composition comprises no more than about 1.0 wt.%, preferably no more than 0.9 wt%, most preferred no more than 0.8 wt % of triptan degradation products after the pharmaceutical composition has been subjected to an accelerated stability test (15 days storage at 60°C).

**4.** The pharmaceutical composition of any of the above claims, wherein the composition comprises no more than about 1.0 %, preferably no more than 0.8 wt %, most preferred below 0.5 wt % of a triptan N-oxide after the pharmaceutical composition has been subjected to an accelerated stability test (15 days storage at 60°C).

**5.** The pharmaceutical composition of any of the above claims, wherein the ratio of the thiol-group containing reducing agent to the triptan is not more than 2:1, in particular not more than 1:1, preferably not more than 0.5:1, and most preferred not more than 0.1:1.

**6.** The pharmaceutical composition of any of the above claims, wherein the thiol-group reducing agent is selected from the group consisting of glutathione, L-cysteine, dithiothreitol, methionine, thioglycerol, thiogycol acid or a pharmaceutically acceptable salt thereof.

**7.** The pharmaceutical composition of any of the above claims, wherein the composition comprises the following components in the given proportions:

a) 20 to 75 wt.% and preferably 30 to 55 wt.% film-forming polymer;
b) 0.02 to 7 wt.% and preferably 0.07 to 6 wt.% thiol-group containing reducing agent, and
c) 2 to 35 wt.% and preferably 5 to 20 wt.% active ingredient.

**8.** The pharmaceutical composition of any of the above claims, wherein the triptan has the formula I as follows:

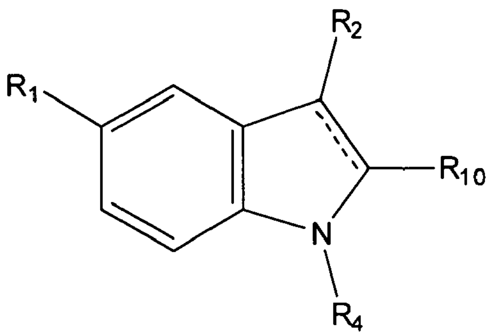

wherein $R_i$ is

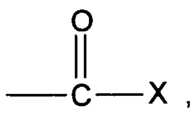

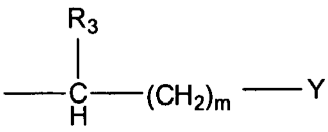
,
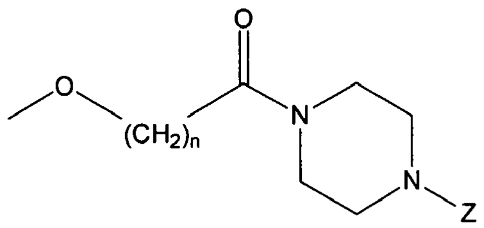

wherein Y is

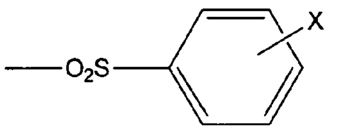
,
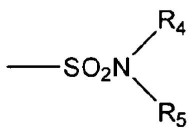

or a 5- or 6-membered cycloalkyl, wherein in some embodiments 1, 2, or 3 .$CH_2$ groups are replaced by 0, S, or NH, which cycloalkyl will in some embodiments substituted with an oxo group;
X is H, C1-3-alkyl, C1-3-alkoxy, halogen, $CF_3$, $N0_2$ or $NH_2$;
$R_3$ is H or C1-3-alkyl;
$R_4$ is H, C1-6-alkyl or C3-6-alkenyl;
$R_5$ is H, C1-3-alkyl, C3-6-alkenyl, aryl, C1-4-alkylene or C5-7-cycloalkyl;
R4 and R5 together form the group $(CR_3)_s$ with s = 2, 3, 4, 5 or 6;
Z is C1-C6 alkyl, C3-C6 alkenyl, C1-4-alkylene or C5-7-cycloalkyl, aryl or heteroaryl,
or Z is

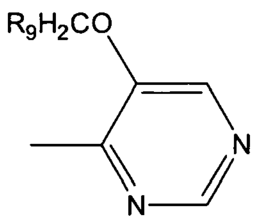
,
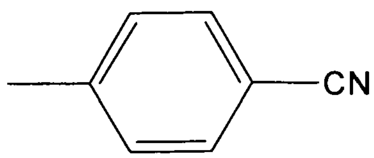

wherein $R_2$ is

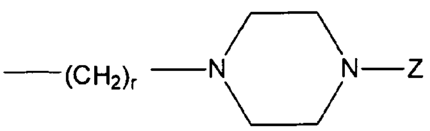
,
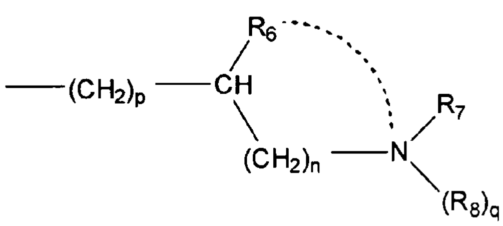

$R_2$ and $R_{10}$ together form the group $(CR_3)_S$ with s = 2, 3, 4, 5 or 6, substituted with $NHR_9$
$R_6$ is H or (CH2)r;
$R_7$ and $R_8$ are the same or different, and are each independently H, or C1-3-alkyl;
$R_9$ is H, C1-6-alkyl, or C3-6-alkenyl;
m, n, and r may be the same or different and are each independently an integer from 0 to 3, e.g., are each independently 0, 1, 2, or 3;

p is an integer that is 0 or 1; and
q is an integer that is 0 or 1;
with the proviso that when $R_6$ is $(CH_2)_r$ and r is not zero, this group can be bound to the nitrogen atom of the radical $NR_7(R_8)_q$ by a single bond, in which case q is zero. In some embodiments, the indole serotonin receptor agonist is a physiologically acceptable salt of a compound of Formula I, or a solvate of a compound of Formula I, or a prodrug of a compound of Formula I. In some embodiments, e.g., the agonist is a succinate salt of a compound of Formula I.

**9.** The pharmaceutical composition of any of the above claims, wherein the triptan is selected from the group consisting of zolmitriptan, sumatriptan, rizatriptan, almotriptan, naratriptan, eletriptan, frovatriptan, avitriptan or donitriptan.

**10.** The pharmaceutical composition of any of the above claims, wherein said pharmaceutical composition is an oral film.

**11.** The pharmaceutical composition of claim 9, wherein the film contains a film forming polymer is a water soluble polymer, preferably a polyvinyl alcohol and more preferably a polyvinyl alcohol of 20-400 KDa and having a degree of hydrolysis of 85 to 90%, and most preferably Mowiol 4-88.

**12.** The pharmaceutical composition of claim 9 or 10, wherein the formulation comprises at least one plasticizer.

**13.** The pharmaceutical composition of claim 11, wherein the plasticizer is a polyethylene glycol polymer (PEG), more preferably a combination of at least one solid PEG and at least one liquid PEG (both at room temperature), most preferably a combination of a PEG with a molecular weight between 3000 and 6000 Da and a PEG with a molecular weight between 250 and 1000 Da, and specifically a combination of PEG 400 and PEG 4000.

**14.** The use of a thiol-group containing reducing agent for the stabilisation of triptans in pharmaceutical compositions.

## Table 1

| Substance | Weight/3 $cm^2$ film [mg/film] | Weight/6 $cm^2$ film [mg/film] | Weight/6 $cm^2$ film [%] | Weighted sample [kg] |
|---|---|---|---|---|
| Zolmitriptan | 2,500 | 5,000 | 10,55 | 3,482 |
| Poly vinyl alcohol (Mowiol 4-88) | 10,500 | 21,000 | 44,31 | 14,622 |
| Macrogol 4000 | 1,500 | 3,000 | 6,33 | 2,089 |
| Macrogol 400 | 0,600 | 1,200 | 2,53 | 0,835 |
| Rice starch | 4,750 | 9,500 | 20,04 | 6,613 |
| Acesulfam K | 0,800 | 1,600 | 3,38 | 1,115 |
| Orange flavour | 1,500 | 3,000 | 6,33 | 2,089 |
| Titan dioxide | 1,500 | 3,000 | 6,33 | 2,089 |
| Glutathione | 0,048 | 0,095 | 0,20 | 0,066 |
| Purified water | | | | 50,250 |
| Ethanol 96% | | | | 16,750 |
| Sum | | | | 100,00 |

## Table 2

| Parameter | Description |
|---|---|
| Column | Xterra RP18 – 250 mm x 4,6 mm, 5 µm (Waters) |
| Column temperature | 30°C |
| Buffer X | Disolve 0,3 g $KH_2PO_4$ and 0,65 $NaH_2PO_4$ x 2 $H_2O$ dissolved in 900 ml Milli-Q® adjust to pH 4.5 ± 0.05 with 5 per cent V/V solution of phosphoric acid and dilute to 1000 mL with water. |
| Mobile phase A | Buffer X / Methanol (80 : 20; v/v) |
| Mobile phase B | Buffer X / Acetonitrile (30 : 70; v/v) |
| Flow rate | 1.0 mL/min |
| Gradient profile | 0 min 100% A<br>0 – 50 min increase to 100% B<br>50 – 55 min increase to 100% A<br>55 – 60 min standby to 100% A<br>60 min Stop |
| Injection volume | 5 µL |
| Detection | UV 225 nm + spectral data |
| Relative retention time | Stage IV RRT approx. 0.31<br>n-Oxid RRT approx. 0.37<br>Amine Impurity RRT approx. 0.59<br>Unknown Impurity RTT0.67 RRT approx. 0.67<br>Zolmitriptan RRT approx. 1.00 |
| Number of Injections | 2 |
| Stop time | 60 min |
| Solvent | Water Milli-Q®/ Acetonitril (50/50) |
| Stock solutions | 3 independent weighings:<br>stock solutions A : Dissolve approx. 10 mg Zolmitriptan base working standard using 10 mL volumetric flask in solvent = SL-ZolmitriptanA.<br>stock solutions B : Dissolve approx. 20 mg Zolmitriptan base working standard using 20 mL volumetric flask in solvent = SL-ZolmitriptanB.<br>stock solutions C : Dissolve approx. 15 mg Zolmitriptan base working standard using 15 mL volumetric flask in solvent = SL-ZolmitriptanA.<br>stock solutions are stable for 2 days, stored at room temperature or in a refrigerator |
| Standard solutions | Std.-A: Dilute 4.0 mL stock solution A to 10 mL using solvent.<br>Std.-B: Dilute 10.0 mL stock solution B to 20 mL using solvent.<br>Std.-C: Dilute 6.0 mL stock solution C to 10 mL using solvent.<br>Std.-0.5%: 2.5 mL Standard B are diluted to 50 mL using solvent = ID 1<br>2.0 mL of this solution ID 1 are diluted to 20 mL using solvent. |
| Sample preparation | Three Rapidfilms are weight independently into a 50 ml Erlenmeyer flasks. Ad 10,0 ml solvent. The solution is shaked for at least 30 minutes at 150 rpm in a horizontal shaker.<br>If the solution is turbidly, centrifuge it for 5 minutes at 4000 rpm.<br>The undeluted sample solution will be inject twice (c Zolmitriptan = 500 µg/ml, dilution = 10).<br>The test solutions can be used within 1 day when stored at room temperature. |

## Table 3

| Stabilising agent [0.2%] | Effect on Zomitriptan stability |
|---|---|
| Tocopherol | reduced stability |
| Sodium EDTA | reduced stability |
| Butylated hydroxytoluene | reduced stability |
| Citric acid | reduced stability |
| Sodium sulfite | reduced stability |
| Ascorbic acid | reduced stability |
| Sodium citrate acid | unchanged stability |
| Ascorbyl palmitate | Slightly increased stability |
| Glutathion | Increased stability |
| L-cysteine | Increased stability |
| L-cysteine-HCl · $H_2O$ | Increased stability |

## Table 4

| Stabilising agent | Zolmitriptan degradation product [% per dried film] | | |
|---|---|---|---|
| | N-Oxid | RTT 0.67 | Total |
| None (batch ZOL047) | 0.91% | 0.18% | 1.71% |
| 02 % glutathione (batch ZOL048) | 0.17% | 0.13% | 0.72% |

**Figure 1**

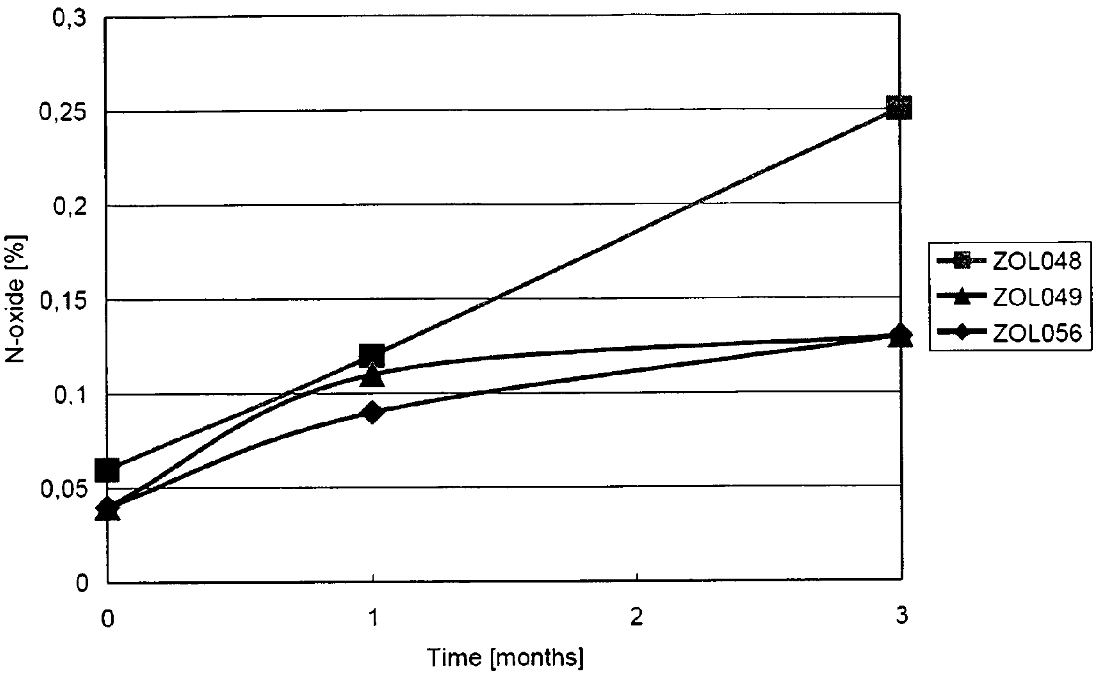
0,3
0,25
0,2
0,15
0,1
0,05
0
N-oxide [%]
0
1
2
3
Time [months]
ZOL048
ZOL049
ZOL056

## Figure 2

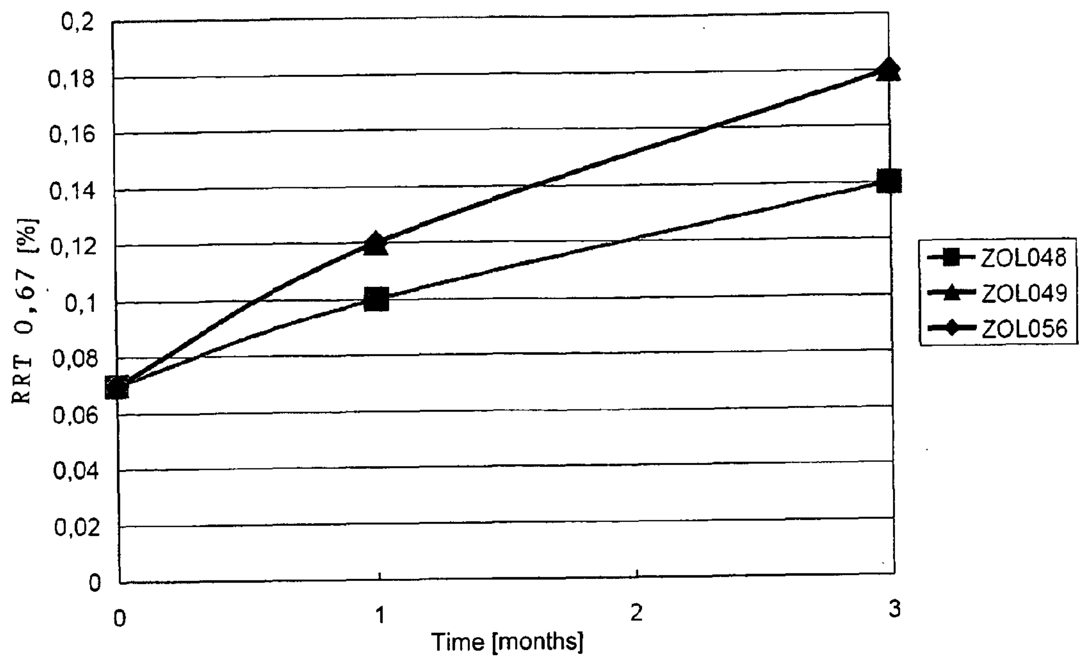
0,2
0,18
0,16
0,14
0,12
0,1
0,08
0,06
0,04
0,02
0
RRT 0,67 [%]
0
1
2
3
Time [months]
ZOL048
ZOL049
ZOL056

Europäisches Patentamt
European Patent Office
Office européen des brevets

# EUROPEAN SEARCH REPORT

Application Number
EP 10 00 3705

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br>Y | WO 2005/120456 A2 (PFIZER LTD [GB]; BENAMEUR HASSAN [FR]; FITZGERALD MICHAEL PAUL [GB]; P)<br>22 December 2005 (2005-12-22)<br>* page 8, line 17 - line 34 *<br>* page 9, line 20 - line 32 *<br>* page 10; example 1; table 2 *<br>* claims 1-11 *<br>----- | 1-6,8,9,<br>12,14<br><br><br>7,10,11,<br>13 | INV.<br>A61K9/00<br>A61K9/70<br>A61K31/422<br>A61K47/18<br>A61K47/20 |
| Y | CN 101 574 330 A (SHANGHAI MODERN PHARMACEUTICAL [CN])<br>11 November 2009 (2009-11-11)<br>* abstract *<br>----- | 7,10,11,<br>13 | |
| A | WO 2007/070504 A2 (MORTON GROVE PHARMACEUTICALS I [US]; DELMARRE DAVID [US]; NARANG AJIT)<br>21 June 2007 (2007-06-21)<br>* the whole document *<br>----- | 1-14 | |
| A,D | WO 2008/040534 A2 (LABTEC GES FUER TECHNOLOGISCHE [DE]; APR APPLIED PHARMA RES S A [CH];) 10 April 2008 (2008-04-10)<br>* the whole document *<br>----- | 1-14 | TECHNICAL FIELDS SEARCHED (IPC)<br>A61K |
| A,D | WO 2009/014960 A1 (TEIKOKU PHARMA USA INC [US]; HIBI TORU [US])<br>29 January 2009 (2009-01-29)<br>* the whole document *<br>----- | 1-14 | |

1

The present search report has been drawn up for all claims

| Place of search | Date of completion of the search | Examiner |
|---|---|---|
| The Hague | 17 August 2010 | Frelichowska, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 00 3705

This annex lists the patent family members relating to the patent documents cited in the above-mentioned European search report.
The members are as contained in the European Patent Office EDP file on
The European Patent Office is in no way liable for these particulars which are merely given for the purpose of information.

17-08-2010

| Patent document cited in search report | Publication date | Patent family member(s) | Publication date |
|---|---|---|---|
| WO 2005120456 A2 | 22-12-2005 | NONE | |
| CN 101574330 A | 11-11-2009 | NONE | |
| WO 2007070504 A2 | 21-06-2007 | NONE | |
| WO 2008040534 A2 | 10-04-2008 | AU 2007304425 A1 | 10-04-2008 |
| | | CA 2664986 A1 | 10-04-2008 |
| | | CN 101626756 A | 13-01-2010 |
| | | EP 2076251 A2 | 08-07-2009 |
| | | KR 20090080037 A | 23-07-2009 |
| | | US 2010173940 A1 | 08-07-2010 |
| WO 2009014960 A1 | 29-01-2009 | AR 067649 A1 | 21-10-2009 |
| | | AU 2008279414 A1 | 29-01-2009 |
| | | CA 2680238 A1 | 29-01-2009 |
| | | CN 101652065 A | 17-02-2010 |
| | | EA 200901188 A1 | 30-04-2010 |
| | | EP 2170063 A1 | 07-04-2010 |
| | | KR 20100020449 A | 22-02-2010 |
| | | US 2009028802 A1 | 29-01-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008040534 A2
- WO 2009014960 A1
- US 3492131 A


### Non-patent literature cited in the description


- Kirk-Othmer Encyclopedia of Chemical Technology. vol. 5, 857-884